# EUROPEAN PATENT SPECIFICATION

(11) **EP 1 190 720 B1**
(45) Date of publication and mention of the grant of the patent: **24.03.2004**
(21) Application number: 00935230.3
(22) Date of filing: 08.06.2000
(51) Int. Cl.: A61K 47/44, A61K 47/38, A61K 47/18, A61K 47/14, A61K 47/26, A61K 31/496, A61K 31/167, A61K 31/7048

(54) **LIQUID PHARMACEUTICAL COMPOSITION FOR ORAL ADMINISTRATION OF BITTER HYDROLYSIS-SUSCEPTIBLE ACTIVE SUBSTANCES**
FLÜSSIGE PHARMAZEUTISCHE ZUSAMMENSETZUNG ZUR ORALEN VERABREICHUNG BITTERSCHMECKENDER HYDROLYSEEMPFINDLICHER AKTIVSTOFFE
COMPOSITION PHARMACEUTIQUE LIQUIDE POUR L'ADMINISTRATION ORALE DE PRINCIPES ACTIFS AMERS ET SUSCEPTIBLES D'HYDROLYSE

(30) Priority: 10.06.1999 ES 9901281
(43) Date of publication of application: 27.03.2002
(73) Proprietor: LABORATORIOS S.A.L.V.A.T., S.A., 08950 Esplugues de Llobregat (Barcelona) (ES)
(72) Inventor: ROIG CARRERAS, Manel, E-08012 Barcelona (ES); TUBAU ARINO, Pere, E-08980 Sant Feliu de Llobregat (ES); JULVE RUBIO, Jordi, E-08758 Cervello (ES)
(74) Representative: SUGRANES - VERDONCES - FERREGÜELA
(86) International application number: PCT/ES2000/000206
(87) International publication number: WO 2000/076549

(56) References cited:
- EP-A- 0 750 849
- WO-A-92/04893
- ES-B- 2 105 970

## Description

This invention refers to the area of techniques for pharmaceutical or galenic compositions or formulations, in particular, for oral administration - not of the extemporaneous type, but of the ready-to-use type - of active pharmaceutical substances that have a bitter or disagreeable taste, and that cannot be in contact with water for a long time as they are susceptible to decomposition via hydrolysis

### BACKGROUND ART

In modern therapeutics, oral administration forms are more desirable than parenteral or rectal administration forms, amongst other reasons because they tend to provoke less rejection in the patients. Amongst the oral forms, the solid forms (tablets, capsules, pills, etc.,) are the most common ones; but their ingestion is often problematic for patients who are little motivated or have difficulties swallowing. Typically, amongst these patients are children, the elderly and those affected with disabilities or acute pain. In these cases, the liquid oral forms, in particular those of ready-to-use type (syrups, elixirs, drops, etc.,) are particularly desirable.

But the liquid oral forms encounter problems when the active substance has a disagreeable taste and/or is unstable in long term contact with water. Also, it is important that the exterior appearance of the composition is homogeneous, therefore the main vehicles of the composition have to be capable of dissolving or suspending all its components, thus, the masking of the taste, the stability and the good dissolution or suspension in the pharmaceutical forms that are liquid and oral, are technical problems to which considerable research and development efforts are currently being directed.

Amongst the liquid formulations proposed in the art for masking the taste of bitter active substances are to be found the suspensions that involve a great amount of sucrose and ingredients included in tablets (starch, lactose, magnesium stearate, etc.,); but these suspensions are usually disagreeable and have to be administered via gastric or nasogastric tubes. In the application WO 9534276 the use of propyleneglycol and sorbitol are proposed for masking the bad taste of some bitter active substances, such as thymol. In order to mask the bad taste of other active substances, such as gabapentin and tacrine, in application WO 9214443-A1 oil-in-water emulsions of microspheres containing the active substance are proposed. But the cited compositions contain water, thus, although useful for extemporaneous suspensions, they are not suitable for ready-to-use suspensions of active substances that are susceptible to decomposition by hydrolysis. WO 9204893-A1 discloses a liquid non-aqueous oral composition in an edible oily vehicle in order to minimize the bitter taste. In ES 2105970-A1 it is proposed to mask the bad taste of ciprofloxacin by the addition of large quantities (around 15 %) of cocoa powder, which implies all the problems and limitations inherent in the ingestion of chocolate. But, in summary, the problem of oral administration, in liquid form, of active substances that are bitter and susceptible to hydrolysis has not yet been solved satisfactorily.

### SUMMARY OF THE INVENTION

The present invention resolves the aforementioned problems by providing a new liquid non aqueous pharmaceutical composition for ready-to-use type oral administration, stable and with improved organoleptic characteristics, which, for every 100 ml, approximately comprises:
- between 3 and 15 g of active substance;
- between 0.01 and 0.8 g of a preservative selected from methyl p-hydroxybenzoate, ethyl p-hydroxybenzoate, propyl p-hydroxybenzoate, butylhydroxyanisole, butylhydroxytoluene, sorbic acid and their mixtures;
- sufficient amount of an edible oily vehicle formed by triglycerides of short- or medium-chain acids for making up the 100 ml;
- a suitable amount of sweetener, optionally accompanied by a suitable amount of flavoring agent;
- between 0.5 and 2 g of soya lecithin;
- between 0.1 and 5 g of ethylcellulose N-50; and
- between 0.05 and 1 g of a surfactant of the polysorbate type.

The presence of lecithin, as illustated in the examples, allows an initial masking of the taste of the active substance, but at the same time acts as an anti-agglomeration agent, facilitating redispersion, and as an emulsifier, improving the speed of dissolution of the active substance and, therefore, its bioavailability.

The derivative of cellulose provides the appropriate viscosity in order to avoid the rapid sedimentation of the incorporated powders, but at the same time contributes to very effectively masking the taste of the active substance, also providing an agreeable and ideal texture for this type of suspension. The special technology for its addition, illustrated in the examples, is important for achieving its efficiency.

The surfactant of polysorbate type favours the speed of dissolution of the composition and, in consequence, the bioavailability of the active substance. In a preferred embodiment, the surfactant of polysorbate type is selected from polysorbate-20, polysorbate-80 and their mixtures.

The edible oily vehicle made up of the triglycerides of short- or medium-chain acids, with a water content lower than 0.5 %, allows the dispersion of a relatively high amount of active substance, at the same time avoiding the phenomena of hydrolysis that occur in the aqueous suspensions. In a preferred embodiment of the invention, the edible oily vehicle made up of the triglycerides of short or medium chain acids is composed of triglycerides of caprylic acid, capric acid or of their mixtures. The preservative guarantees the physico-chemical stability and avoids the microbiological contamination of the composition.

The presence of the sweetener, optionally accompanied by the flavoring agent, finishes off the masking of the repugnant taste of the active substance, in a way that is perfectly bearable to the palate. In a preferred embodiment of the invention, the appropriate amount of sweetener is selected from: 0.01-0.2 g of sodium saccharin, 0.1-5 g of sodium cyclamate, 0.1-2 g of ammonium glycyrricinate and their mixtures. In a particular embodiment, the appropriate amount of flavoring agent is 0.1-0.5 g of one or various of the following flavours: walnut, condensed milk, coconut and banana.

In a particularly preferred embodiment, the composition also contains 0.1-5 g of mannitol, that apparently helps the solubility of the composition in the stomach.

In another particularly preferred embodiment, the composition also contains 1-5 g of sodium bicarbonate which, upon reacting with the acid medium of the stomach and forming carbon dioxide, causes an effervescence that facilitates the release of the active substance and its posterior dissolution.

In preferred embodiments of the present invention, illustrated in the enclosed examples, the active substance is selected from ciprofloxacin, paracetamol and erythromycin. The composition with ciprofloxacin is especially preferred.

The compositions of the present invention prove to be surprisingly stable for long periods of time, which permits their use as a suspension for ready-to-use administration, whose advantages with respect to extemporaneous administration are well known.

The oily suspension of the invention provides a good bioavailability of the active substance, which translates into a high efficiency shortly after administration. As the taste proves to be acceptable, its administration is advantageous with respect to the known solid forms, especially in children and patients with little motivation.

### DETAILED DESCRIPTION OF A PREFERRED EMBODIMENT

The following examples illustrate the invention for the particular cases of three active substances of disagreeable taste, namely: ciprofloxacin, paracetamol and erythromycin. The final amount of water in all the compositions is of less than 0.5 %.

### Example 1: General preparation process of the liquid compositions

For preparing a total amount of 100 ml of suspension, the following operations are followed:
a) Coating of the bitter active substance (between 3 and 15 g) with a concentrated lecithin solution (from 0.5 to 2 g of dry residue).
b) Drying (between 35°C and 45°C) and granulation.
c) Pulverisation of the granules obtained in (b), to a particle size of between 150 and 250 micras.
d) Coating of the powder obtained in (c) with mannitol (0.1-0.5 g) and polysorbate 20, polysorbate 80 or their mixtures (0.1-0.5 g).
e) Drying (between 35°C and 45°C) and granulation.
f) Pulverisation of the granules obtained in (e), to a particle size of between 100 and 150 micras.
g) Addition, under continuous stirring, of ethylcellulose N-50 (1 to 5 g) to a substantial part of the total volume of the mixture of triglycerides of short- and medium-chain acids, previously heating slightly above 100°C.
h) Cooling to 35-40°C of the mixture obtained in (g), and addition, with continuous stirring, of the powder obtained in (f).
i) Optional addition, with continuous stirring, of the sodium bicarbonate (1-5 g).
j) Addition, with continuous stirring, of the preservative (sorbic acid, methyl or ethyl p-hydroxybenzoate, butylhydroxyanisol, butylhydroxytoluene; 0.1- 0.8 g).
k) Standing (e.g. 10-18 h).
1) Addition, with continuous stirring, of the sweetener (sodium cyclamate, sodium saccharin, ammonium glycyrricinate; 0.1-5 g).
m) Addition, with continuous stirring, of the flavoring agent (walnut flavouring, condensed milk flavouring, coconut flavouring, banana flavouring; 0.1-0.5 g).
n) Addition, with continuous stirring, of the remaining volume of the triglycerides of short- and medium-chain acids in order to complete the 100 ml of suspension.

### Example 2: Liquid composition of milk and walnut flavoured ciprofloxacin

Following the general technique described in Example 1, various suspensions of ciprofloxacin were prepared with the compositions indicated (amounts in grams per 100 ml), which obtained a good organoleptic acceptation in tests on healthy volunteers:

| | |
|---|---|
| Ciprofloxacin | 5-15 |
| Soya lecithin | 0.5-2.0 |
| Mannitol | 0.5-5.0 |
| Polysorbate 20 | 0.05-1.0 |
| Ethylcellulose N-50 | 0.1-5.0 |
| Ammonium glycyrricinate | 0.1-2.0 |
| Sodium cyclamate | 0.1-5.0 |
| Sodium saccharin | 0.01-0.2 |
| Sorbic acid | 0.01-0.3 |
| Walnut flavouring | 0.1-0.5 |
| Condensed milk flavouring | 0.1-0.5 |
| Sodium bicarbonate | 1-5 |
| Medium chain triglycerides (Estasan™3775) | q.s.100 ml |

### Example 3: Liquid composition of banana flavoured paracetamol

Following the general technique described in Example 1, various suspensions of paracetamol were prepared with the compositions indicated (amounts in grams per 100 ml), which obtained a good organoleptic acceptation in tests on healthy volunteers:

| | |
|---|---|
| Paracetamol | 5-10 |
| Soya lecithin | 0.5-2.0 |
| Mannitol | 0.5-2.0 |
| Polysorbate 20 | 0.05-1.0 |
| Ethylcellulose N-50 | 0.1-5.0 |
| Sodium cyclamate | 0,1-5,0 |
| Sorbic acid | 0.01-0.3 |
| Banana flavouring | 0.1-0.5 |

Medium chain triglycerides (Estasan™3775) q.s. 100 ml

### Example 4: Liquid composition of milk flavoured erythromycin

Following the general technique described in Example 1, various suspensions of erythromycin were prepared with the compositions indicated (quantities in grams per 100 ml), which obtained a good organoleptic acceptation in tests on healthy volunteers:

| | |
|---|---|
| Erythromycin | 3-6 |
| Soya lecithin | 0.5-2.0 |
| Polysorbate 80 | 0.05-1.0 |
| Ethylcellulose N-50 | 0.1-5.0 |
| Sodium cyclamate | 0.1-5.0 |
| Sorbic acid | 0.01-0.3 |
| Condensed milk flavouring | 0.1-0.5 |
| Sodium bicarbonate | 1-5 |
| Medium chain triglycerides (Estasan™3775) | q.s. 100 ml |

## Claims

1. Liquid non-aqueous pharmaceutical composition for ready-to-use type administration, stable and with improved organoleptic characteristics, which, per 100 ml, contain: between 3 and 15 g of active substance;
between 0.01 and 0.8 g of a preservative selected from methyl p-hydroxybenzoate, ethyl p-hydroxybenzoate, propyl p-hydroxybenzoate, butylhydroxyanisole, butylhydroxytoluene, sorbic acid and their mixtures; between 0.5 and 2 g of soya lecithin; between 0.1 and 5 g of ethylcellulose N-50; between 0.05 and 1 g of a polysorbate type surfactant; a suitable amount of sweetener, optionally accompanied by a suitable amount of flavoring agent; and sufficient amount of an edible oily vehicle formed by triglycerides of short- or medium-chain acids for making up the 100 ml.

2. Composition according to any of the preceding claims, wherein the polysorbate type surfactant is selected from polysorbate-20, polysorbate-80, and their mixtures.

3. Composition according to any of the preceding claims, wherein the edible oily vehicle made up of triglycerides of short- or medium-chain acids is composed of triglycerides of caprylic acid, of capric acid, or of their mixtures.

4. Composition according to any of the preceding claims, wherein the appropriate amount of sweetener is selected from: 0.01-0.2 g of sodium saccharin, 0.1-5 g of sodium cyclamate, 0.1-2 g of ammonium glycyrricinate, and their mixtures.

5. Composition according to any of the preceding claims, wherein the appropriate amount of the flavoring agent is 0.1-0.5 g and the flavour is selected from one or more of the following : walnut, condensed milk, coconut and banana.

6. Composition according to any of the preceding claims, which further includes 0.1-5 g of mannitol.

7. Composition according to any of the preceding claims, which further includes 1-5 g of sodium bicarbonate.

8. Composition according to any of the preceding claims, wherein the active substance is selected from ciprofloxacin, paracetamol and erythromycin.

9. Composition according to any of the preceding claims, wherein the active substance is ciprofloxacin.

## Revendications

1. Composition pharmaceutique liquide non aqueuse pour administration type prête à l'emploi, stable et avec des caractéristiques organoleptiques améliorées, qui contient pour 100 ml : entre 3 et 15 g du composé actif ; entre 0,01 et 0,8 g d'un conservateur sélectionné parmi le méthyle p-hydroxybenzoate, l'éthyle p-hydroxybenzoate, le propyle p-hydroxybenzoate, le butyle hydroxyanisol, le butylhydroxytoluène, l'acide sorbique et leurs mélanges ; entre 0,5 et 2 g de lécithine de soja ; entre 0,1 et 5 g d'éthyle cellulose N-50 ; entre 0,05 et 1 g de tensioactif type polysorbate ; une quantité appropriée d'édulcorant, accompagnée en option d'une quantité appropriée d'agent aromatisant ; et une quantité suffisante d'un véhicule huileux comestible formé par des triglycérides d'acides à chaîne courte et moyenne pour compléter jusqu'aux 100 ml.

2. Composition selon la revendication précédante, où le tensioactif type polysorbate est sélectionné parmi le polysorbate-20, le polysorbate-80 et leurs mélanges.

3. Composition selon n'importe quelle des revendications précédantes, où le véhicule huileux comestible fait de triglycérides d'acides à chaînes courte et moyenne se compose de triglycérides de l'acide caprylique, de l'acide caprique ou de leurs mélanges.

4. Composition selon n'importe quelle des revendications précédantes où la quantité appropriée d'édulcorant est sélectionnée parmi : 0,01-0,2 g de saccharine sodique, 0,1-5 g de cyclamate de sodium, 0,1-2 g de glycinate de ricin ammoniac et leurs mélanges.

5. Composition selon n'importe quelle des revendications précédantes, où la quantité appropriée d'agent aromatisant est comprise entre 0,1-1,0 g et l'arôme est sélectionné parmi un ou plusieurs des arômes suivants : noix, lait concentré sucré, noix de coco et banane.

6. Composition selon n'importe quelle des revendications précédantes qui inclut en plus 0,1-5 g de mannitol.

7. Composition selon n'importe quelle des revendications précédantes qui inclut en plus 1-5 g de bicarbonate sodique.

8. Composition selon n'importe quelle des revendications précédantes où le composé actif est sélectionné parmi la ciprofloxacine, le paracétamol et l'érythromycine.

9. Composition selon n'importe quelle des revendications précédantes où le composé actif est la ciprofloxacine.

## Patentansprüche

1. Flüssige, nicht wässrige pharmazeutische Verbindung für die sofortige Verabreichung, stabil und mit verbesserten organoleptischen Merkmalen, die pro 100 ml enthält: zwischen 3 und 15 g aktive Substanz; zwischen 0.01 und 0.8 g eines Konservierungsstoffes, der ausgewählt wird aus Methyl-p-Hydroxybenzoat, Ethyl-p-Hydroxybenzoat, Propyl-p-Hydroxybenzoat, Butylhydroxyanisol, Butylhydroxytoluen, Sorbinsäure und deren Mischungen; zwischen 0.5 und 2 g Soja-Lecithin; zwischen 0.1 und 5 g Ethylcellulose N-50; zwischen 0.05 und 1 g oberflächenaktives Polysorbat; eine geeignete Menge Süßstoff, optional begleitet von einer geeigneten Menge Aromastoff; und eine hinreichende Menge an essbarem ölhaltigern Bindemittel aus Triglyceriden aus kurz- oder mittelkettigen Säuren bis auf 100 ml.

2. Zusammensetzung nach irgendeinem der vorstehenden Ansprüche, wobei das oberflächenaktive Polysorbat aus Polysorbat-20, Polysorbat-80 und deren Mischungen ausgewählt wird.

3. Zusammensetzung nach irgendeinem der vorstehenden Ansprüche, wobei das essbare ölhaltige Bindemittel aus Triglyceriden aus kurz- oder mittelkettigen Säuren aus Kaprylsäure, Kaprinsäure oder deren Mischungen besteht.

4. Zusammensetzung nach irgendeinem der vorstehenden Ansprüche, wobei die geeignete Menge an Süßstoff ausgewählt wird aus: 0.01-0.2 g Natriumsaccharin, 0.1-5 g Natriumcyclamat, 0.1-2 g Ammoniumglycyrricinat und deren Mischungen.

5. Zusammensetzung nach irgendeinem der vorstehenden Ansprüche, wobei die geeignete Menge an Aromastoff 0.1-1.0 g entspricht und der Aromastoff aus einem oder mehreren der folgenden ausgewählt wird: Walnuss, Kondensmilch, Kokosnuss und Banane.

6. Zusammensetzung nach irgendeinem der vorstehenden Ansprüche, wobei zusätzlich 0.1-5 g Mannitol enthalten sind.

7. Zusammensetzung nach irgendeinem der vorstehenden Ansprüche, wobei zusätzlich 1-5 g Natriumbicarbonat enthalten sind.

8. Zusammensetzung nach irgendeinem der vorstehenden Ansprüche, wobei die aktive Substanz ausgewählt wird aus Ciprofloxazin, Paracetamol und Erythromycin.

9. Zusammensetzung nach irgendeinem der vorstehenden Ansprüche, wobei die aktive Substanz Ciprofloxazin ist.
